(19) Europäisches Patentamt
European Patent Office
Office européen des brevets

(11) **EP 3 202 436 B1**

(12) **EUROPEAN PATENT SPECIFICATION**

(45) Date of publication and mention
of the grant of the patent:
**18.12.2019 Bulletin 2019/51**

(21) Application number: **15846367.9**

(22) Date of filing: **16.09.2015**

(51) Int Cl.:
***A61M 1/18*** *(2006.01)* ***A61M 1/14*** *(2006.01)*

(86) International application number:
**PCT/JP2015/076326**

(87) International publication number:
**WO 2016/052206 (07.04.2016 Gazette 2016/14)**

(54) **ARTIFICIAL LUNG AND ARTIFICIAL HEART-LUNG CIRCUIT DEVICE**

KÜNSTLICHE LUNGE UND KÜNSTLICHE HERZ-LUNGEN-KREISLAUFVORRICHTUNG

POUMON ARTIFICIEL ET DISPOSITIF À CIRCUIT C UR-POUMON ARTIFICIEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2014 JP 2014201781**

(43) Date of publication of application:
**09.08.2017 Bulletin 2017/32**

(73) Proprietor: **Terumo Kabushiki Kaisha
Tokyo 151-0072 (JP)**

(72) Inventors:
• **SAITO, Takashi
Elkton, Maryland 21921 (US)**
• **SASAKI, Eisuke
Elkton, Maryland 21921 (US)**

(74) Representative: **Casalonga
Casalonga & Partners
Bayerstraße 71/73
80335 München (DE)**

(56) References cited:
**WO-A1-2012/133372     JP-A- H06 237 992
JP-A- H06 237 992     JP-A- H06 237 993
JP-A- 2003 520 617     US-A- 5 706 889
US-A1- 2014 030 146**

## Description

Technical Field

The present invention relates to design and performance evaluation of an artificial lung.

Background of the Invention

An artificial lung is an artificial organ which supplies oxygen to blood and removes carbon dioxide gas from blood, to replace or supplement the functions of a biological lung. Currently, an external circulation-type of artificial lung or oxygenator using a hollow fiber membrane is mainly adopted. Typically, the artificial lung can be used for up to several hours to support heart surgery during which the heart is stopped, wherein a so-called cardiotomy is used for forming an extracorporeal blood circulation circuit while the biological lung is typically not perfused. In addition, the artificial lung can also be utilized as a respiration assisting apparatus or a percutaneous cardiopulmonary assisting apparatus for a patient of acute pulmonary insufficiency or cardiac insufficiency.

In the artificial lung used in extracorporeal circulation of blood, since blood is guided out of the body of a patient, heat is taken from the blood. Therefore, when blood is returned to the patient, the blood is required to be rewarmed to be near the body temperature. In addition, a conventional extracorporeal circulation method sometimes employs a technique wherein the blood temperature is lowered so as to restrain metabolism of cells while the brain and central nerves are protected. Therefore, the artificial lung is normally provided with a heat exchanging element for adjusting a blood temperature.

The extracorporeal circulation circuit such as an artificial heart-lung circuit device is normally prepared for use by being filled with a priming solution or the like. A lactate Ringer solution is used as the priming solution, and blood becomes diluted by a corresponding filling amount (blood filling amount) of a circuit. The diluted blood leads to a reduced effectiveness and corresponding a burden to a patient. In order to reduce the effects of diluted blood, a blood transfusion can be performed. However, the blood transfusion carries a risk of a physical burden or an infection occurring in a patient. Therefore, a reduction of the blood filling amount of the extracorporeal circulation circuit is desired. As a result thereof, in the artificial lung which is one of the components of the extracorporeal circulation circuits, reduction of the blood filling amount is desirable for the artificial lung as well.

In the design and performance evaluation of an artificial lung, it is favorable to have gas exchange performance of the artificial lung and heat exchange performance of the artificial lung to be as high as possible, and it is favorable to have the blood filling amount of the artificial lung and a blood side pressure loss of the artificial lung to be as low as possible.

For example, in a case where the blood filling amount is reduced so as to minimize a burden to a patient, improvements such as reducing the surface area of a gas exchanging hollow fiber membrane or the heat exchanging element and acquiring high performance through a small surface area by densely installing the gas exchanging hollow fiber membrane or the heat exchanging element are may be required.

However, when the surface area of the gas exchanging hollow fiber membrane or the heat exchanging element is reduced, the gas exchange performance and the heat exchange performance thereof are deteriorated. When the gas exchanging hollow fiber membrane or the heat exchanging element are densely packed, there is concern of an increase of a blood side pressure loss and an increase of damage to the blood cells.

In the related prior art, each of the elements of the artificial lung has been improved independently from other elements (PTL 1), and there is a demand for a technology in which such various elements are united and the elements can be evaluated and examined regardless of the type and the size of the artificial lung.

Citation List

Patent Literature

[PTL 1] JP-T-2014-514963
Further relevant prior art is disclosed in documents US2014/030146 A1, JP H06 237992 A and US5706 889 A.

Summary of Invention

Technical Problem

There is provided a technology of designing and evaluating an artificial lung in which performance of each of elements of the artificial lung can be uniformly examined and a generally excellent performance can be exhibited, and there is provided an artificial lung exhibiting the generally excellent performance acquired by using this method.

Solution to Problem

The inventors have found that regardless of the difference of the type and the structure of the details of an artificial lung, when gas exchange performance, a heat exchange performance coefficient, a blood filling amount, and a blood side pressure loss are measured, performance factors of the artificial lung can be acquired through combinations of the measured results, and regardless of the type and the structure thereof, the artificial lung can be generally evaluated and compared through the acquired performance factors. Thus, the present invention has been realized.

In other words, below is provided according to the present invention.

(1) An artificial lung including a gas exchanger portion that is provided with an external circulation-type gas exchanging hollow fiber membrane bundle, and a heat exchanger portion that is provided with at least a heat exchanging element. Gas exchange performance of the artificial lung, a blood filling amount of the artificial lung, and a blood side pressure loss of the artificial lung are measured, and when Fp1=gas exchange performance of artificial lung/(blood filling amount of artificial lung×blood side pressure loss of artificial lung) is calculated, a value of Fp1 is equal to or greater than 2.5:

> As used herein, gas exchange performance of the artificial lung can be derived according to a method disclosed in Artificial Organ 16(1), 654-657 (1987), "Examination of Artificial Lung Performance Evaluation Method" by Nogawa, wherein the gas exchange performance is preferably determined in the unit [L/min].
> As used herein, Blood filling amount of the artificial lung is measured in the unit [mL] (1 mL=1 L/1,000) conforming to ISO 7199 5.3.3, and
> blood side pressure loss of the artificial lung is measured in the unit [mmHg/(L/min)].

(2) An artificial lung including a gas exchanger portion that is provided with the external circulation-type gas exchanging hollow fiber membrane bundle, and a heat exchanger portion that is provided with at least a heat exchanging element. Gas exchange performance of the artificial lung, a heat exchange performance coefficient of the artificial lung, a blood filling amount of the artificial lung, and a blood side pressure loss of the artificial lung are measured, and when Fp= (gas exchange performance of artificial lung×heat exchange performance coefficient of artificial lung)/(blood filling amount of artificial lung×blood side pressure loss of artificial lung) is calculated, a value of Fp ranges from 1.5 to 2.5:

> As used herein, gas exchange performance of the artificial lung, the blood filling amount of the artificial lung, and the blood side pressure loss of the artificial lung are the same as those described above, and
> the heat exchange performance coefficient of the artificial lung is comprised of a dimensionless (i.e., scalar or absolute) number in accordance with ISO 7199 5.4.2.

(3) An artificial lung including a gas exchanger portion that is provided with an external circulation-type gas exchanging hollow fiber membrane bundle. Gas exchange performance of the gas exchanger portion, a blood filling amount of the gas exchanger portion, and a blood side pressure loss of the gas exchanger portion are measured, and when Fp2=gas exchange performance of gas exchanger portion/ (blood filling amount of gas exchanger portion×blood side pressure loss of gas exchanger portion) is calculated, a value of Fp2 is equal to or greater than 10:

> The gas exchange performance of the gas exchanger portion can be derived according to a method disclosed in Artificial Organ 16(1), 654-657 (1987), "Examination of Artificial Lung Performance Evaluation Method" by Nogawa, wherein the gas exchange performance is preferably determined in the unit [L/min] .
> As used herein, blood filling amount of gas exchanger portion is measured in the unit [mL] (1 mL=1 L/1,000) and is measured based on the volume of a region where the hollow fiber membrane bundle and blood come into contact with each other.
> As used herein, blood side pressure loss of the gas exchanger portion is measured in the unit [mmHg/(L/min)].

(4) The artificial lung according to any one of (1) to (3) . The gas exchange performance is a blood flow rate at which blood having oxygen saturation of 97.5% can be acquired in a case where the artificial lung is operated with hemoglobin concentration of 12.0 g/dL and oxygen saturation of 50% in venous blood.

(5) An artificial lung including the gas exchanger portion according to (3), and a heat exchanger portion.The gas exchanger portion can be combined with an arbitrary heat exchanger portion so as to form the artificial lung, a known heat exchanger portion may be adopted, or a heat exchanger portion having new structure may be adopted.

(6) An artificial heart-lung circuit device including the artificial lung according to any one of (1) to (4).

Advantageous Effect of Invention

In the artificial lung according to the present invention, regardless of the type and the difference of the structure of the details of the artificial lung, evaluation can be generally performed by using performance factors of Fp, Fp1, and Fp2, and thus, a generally excellent artificial lung performance can be acquired.

Brief Description of Drawings

[Fig. 1] Figs. 1A and 1B are cross-sectional views illustrating examples of structure of artificial lungs.
[Fig. 2] Fig. 2 is a schematic view describing an artificial heart-lung circuit device.

Detailed Description of Preferred Embodiments

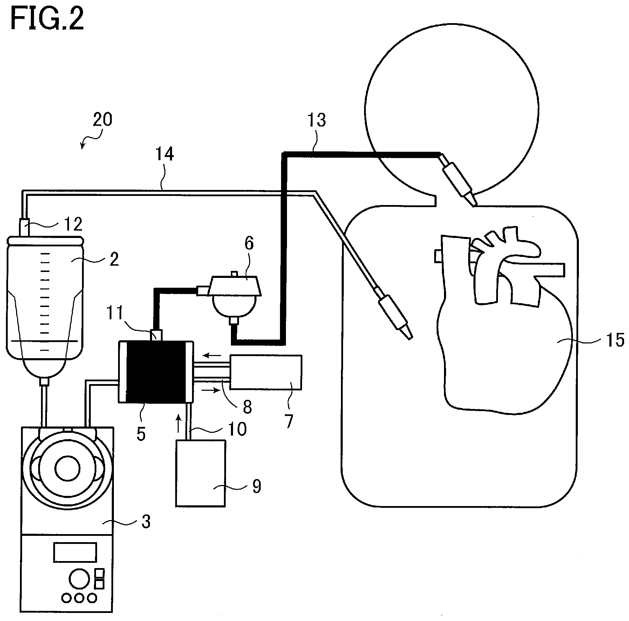

First, description will be given regarding an artificial heart-lung circuit device 20 of the present invention illustrated in Fig. 2. Fig. 2 is a conceptual circuit diagram of an example of an artificial heart-lung circuit device of the present invention and illustrates an artificial lung in which a heat exchanging element is internally mounted. A heat exchanger portion (not illustrated) that is provided with the heat exchanging element and a gas exchanger portion (not illustrated) that is provided with a hollow fiber membrane bundle are included within an artificial lung 5. The artificial heart-lung perfusion circuit 20 includes a venous blood reservoir 2 provided with a blood retention portion which retains blood and from which blood inside thereof can flow out by a blood supply pump 3. The artificial lung 5 connected to a blood outlet port of the blood supply pump 3, and an artery filter 6 is provided on a downstream side of the artificial lung 5. A heat medium supplier 7 is provided which supplies a heat medium to the heat exchanger portion of the artificial lung 5, and a heat medium 8 from the heat medium supplier 7 circulates in the artificial lung 5, thereby performing heat-exchanging. In addition, oxygen-containing gas 10 is supplied from an oxygen-containing gas supplier 9 to the inside of the hollow fiber membrane bundle of the artificial lung 5.

Specifically, the artificial heart-lung perfusion circuit 20 interconnects the vein reservoir 2, a blood removal (venous blood) line 14 which is connected to a blood inlet port 12 of the vein reservoir 2 and through which blood removed from a heart 15 is received, and a blood supply (arterial blood) line 13 through which blood is supplied to the heart 15 through a blood outlet port 11 of the artificial lung 5 via the artery filter 6.

<Performance Factor of Artificial Lung in Entirety>

According to the present invention, in evaluation of an artificial lung device, it is favorable to consider various factors, such as, gas exchange performance of artificial lung [measured in L/min] and a heat exchange performance coefficient of artificial lung [measured as a dimensionless, scalar number], which are preferably made as large as possible. Furthermore, it is desirable to have a blood filling amount of artificial lung [measured in mL] (1 mL=1 L/1,000) and a blood side pressure loss of artificial lung [measured in mmHg/(L/min)] to be as small as possible. In order to optimize performance of an artificial lung, the present invention defines a performance factor Fp of the gas exchanger portion and the heat exchanger portion of the artificial lung through the following Expression:

```
Fp=(gas   exchange   performance   of   artificial   lung [L/min]×heat exchange performance coefficient of artificial

lung [absolute number])/(blood filling amount of artificial lung  [L]×blood  side  pressure  loss  of  artificial  lung

[mmHg/(L/min)]).
```

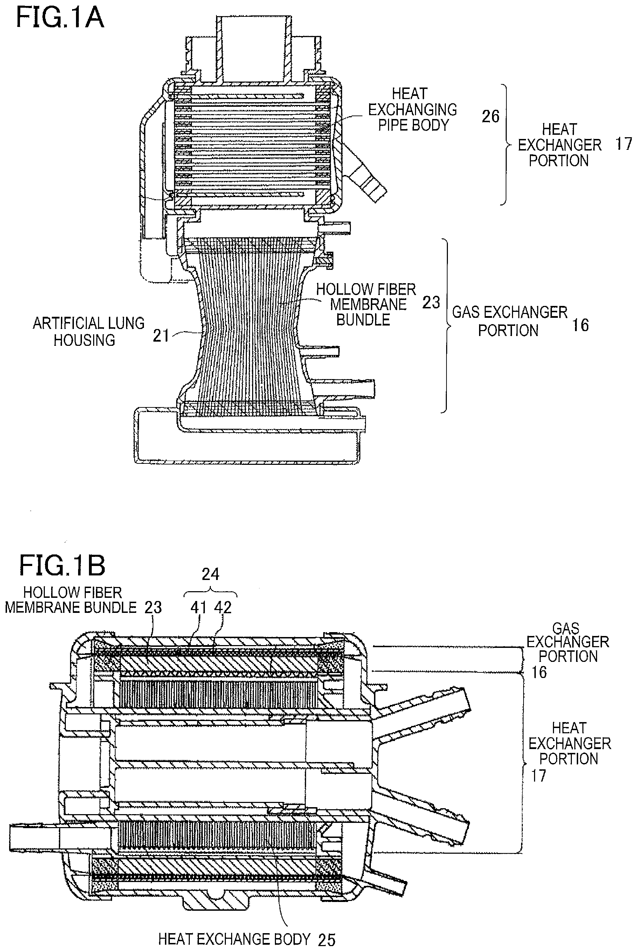

Fig. 1 illustrates cross-sectional views of two examples of external circulation-type artificial lungs in which hollow fiber membranes are used. In the artificial lung illustrated in Fig. 1 [Fig. 1A], a tubular body-shaped artificial lung housing 21 is formed and a hollow fiber membrane bundle 23 is internally accommodated, thereby configuring a gas exchanger portion 16. Above the gas exchanger portion 16, there is provided a heat exchanger portion 17 in which multiple heat exchanging pipe bodies 26 serving as the heat exchanging elements are disposed and blood flows in the heat exchanging pipe bodies 26 and is subjected to heat exchange.

In the artificial lung illustrated in Fig. 1 [Fig. 1B], a housing in its entirety has a cylindrical shape. A filter member 41 configure air bubble removal means 24 which functions as an artery filter internally provided in a concentric cylindrical manner from a side close to the side wall of the housing, and the hollow fiber membrane bundle 23 is internally disposed in a concentric circle manner, thereby configuring the gas exchanger portion 16. An element heat exchange body 25 for heat exchanging is disposed further inside, thereby configuring the heat exchanger portion 17.

As described in the examples of Fig. 1, the artificial lung of the present invention is provided with at least the gas exchanger portion 16 and the heat exchanger portion 17. Examples of the gas exchanger portion include a region which is configured to have the gas exchanging hollow fiber membrane bundle 23 disposed in the artificial lung 5, and an external circulation blood portion surrounding the hollow fiber membrane bundle 23 and allowing blood to flow therein, and in which the hollow fiber membrane bundle 23 and the blood come into contact with each other. The heat exchanger portion 17 is configured to have the heat exchanging element and a heat medium circulation chamber surrounding the heat exchanging element.

Description will be given regarding a method of measuring each of the factors for calculating a performance factor for optimizing performance of the artificial lung as a whole.

1) Gas exchange performance of artificial lung can be measured in the units of [L/min] according to the method described in Artificial Organ 16(1), 654-657 (1987), "Examination of Artificial Lung Performance Evaluation Method" by Nogawa.
Below is the contents disclosed in "Examination of Artificial Lung Performance Evaluation Method".

1-a) An artificial lung is considered to be a black box. Examples of inputs to the artificial lung include Sv: the rate of oxygenated hemoglobin in venous blood, Hb: the hemoglobin concentration of test blood, and QB: the blood flow rate. As an output from the artificial lung, $S_AO2$: the rate of oxygenated hemoglobin of the blood supply line can be examined. When the output with respect to all of the inputs is measured, the gas exchange performance of the artificial lung can be evaluated. However, it has been empirically learned that the conditions are not complete independent variables and are correlated to each other. Therefore, the evaluation can be simplified by reducing the independent variables.

1-b) In consideration of the quantity of $Hb \times QB \times (1-SvO2)$, the quantity is a quantity which can be referred to as the oxygen vacancy quantity (the quantity of oxygen which can be received) with respect to diffusion. When the quantities are equal to each other between different types of artificial lungs, it is assumed that the diffusion states in the artificial lungs are similar to each other (called a diffusion stratum similarity theory).

1-c) Since the above-referenced theory is broadened to a turbulence-type artificial lung, the performance is improved due to an increase of the flow rate and an increase of the Reynold's number. Therefore, the phenomenon is described based on a varying diffusion coefficient D. Since Hb and Re have a positive correlationship, a correction coefficient of Re is taken in both Hb and QB, thereby acquiring a grounded theoretical expression broadened to the turbulence-type artificial lung.

1-d) According to ISO 7199 5.4.1, (1-SAO2)=97.5% and Hb=12 g/dl,
X-axis: $(1-SvO2) \times Hb \times QB$, and Y-axis: (1-SvO2) are established. Here, Since (1-SvO2) is determined by the Y-axis, (1-SvO2) is taken from the X-axis. Furthermore, the index of QB is removed. As a result thereof, a curve having the X-axis: (1-SvO2) and the Y-axis: QB is acquired.

1-e) It is extremely difficult to perform an experiment for drawing a graph of 1-d). However, since experimental data of (1-SAO2)=97.5% can be calculated through an experiment other than (1-SAO2)=97.5% by using a variable transformation method, a graph (not illustrated) can be drawn. The variable transformation method used here is a method in which in a case where a value expressed by a complex function f(x) is unknown, a variable acquired through the experiment other than (1-SAO2)=97.5% transformed to a variable of the experimental data of (1-SAO2)=97.5% such that x (unknown variable) of the function f(x) does not change.

In other words, the gas exchange performance of the artificial lung is defined as a blood flow rate at which oxygen saturation of blood flowing out when blood having the hemoglobin concentration of 12.0 g/dL and the oxygen saturation of 50% flows in the artificial lung becomes 97.5%. As the gas exchange performance of the artificial lung increases, the value of the blood flow rate becomes greater. However, it is difficult to acquire the blood flow rate through an experiment. The reason is that in the calculation of the gas exchange performance, the oxygen saturation on the outlet side (artery side) is an input and the blood flow rate is an output. Meanwhile, in an experiment, the oxygen saturation on the outlet side (artery side) becomes an output and the blood flow rate becomes an input. Thus, by using the variable transformation method disclosed in Artificial Organ 16(1), 654-657 (1987), "Examination of Artificial Lung Performance Evaluation Method" by Nogawa, it is possible to acquire the blood flow rate from several pieces of experimental data, that is, the value of the gas exchange performance. Artificial Organ 16(1), 654-657 (1987), "Examination of Artificial Lung Performance Evaluation Method" by Nogawa can be referred to for more details of the variable transformation method.

A method of measuring gas exchange performance of the present invention will be specifically described. The artificial lung is embedded in an appropriate test circuit, and oxygen gas is supplied to the artificial lung from an oxygen gas supplier. Blood is caused to flow into the artificial lung under conditions of arbitrary oxygen saturation and an arbitrary blood flow rate on an inlet side (vein side), and blood at the entrance and the exit of the artificial lung is taken, thereby acquiring the oxygen saturation. It is desirable that the hemoglobin concentration is $12.0\pm1$ g/dL. It is desirable to set the conditions of the oxygen saturation and the blood flow rate on the inlet side (vein side) such that the oxygen saturation on the outlet side (artery side) does not exceed 99%.

By using the acquired value and the variable transformation method disclosed in Artificial Organ 16(1), 654-657 (1987), "Examination of Artificial Lung Performance Evaluation Method" by Nogawa, a blood flow rate QB is calculated so as to acquire a result having the hemoglobin concentration Hb of 12.0 g/dL, the oxygen saturation SvO2 of 50% in venous blood, and the oxygen saturation $S_AO2$ of 97 .5% in arterial blood. The blood flow rate QB thereof corresponds to the gas exchange performance.

Specifically, measurement under several conditions is performed in this experiment, and the measurement results are evaluated according to,

[Math 1]

$$S_AO2/S_vO2 = f\{(1-S_vO2)\times Hb^{(1+cRe)} \times QB^{(1-cRe-cQB)}\} \quad \ldots \text{Expression (1)}$$

Using substitution for cRe and cQB, and wherein $S_AO2=0.975$ (that is, 97.5%)

SvO2=0.500 (that is, 50%), and

Hb=12.0

, QB can be obtained. The QB is the gas exchange performance of the present invention.

2) Heat exchange performance coefficient of the artificial lung can be measured as an absolute, scalar number using the method of ISO 7199 5.4.2

Thus, a heat exchange performance coefficient R is determined as follows. R=[(temperature Bout of blood at the blood outlet port of artificial lung) - (temperature Bin of blood at blood inlet port of artificial lung)]/[(temperature Win of heat medium at heat medium inlet port of heat exchanger portion of artificial lung)-(temperature Bin of blood at blood inlet port of artificial lung)]

As a test solution for a blood flow path, blood which is taken from a cow and is subjected to heparinization is used. A test is performed under conditions in which the temperature Bin of blood at the blood inlet port of the artificial lung is $30\pm1$°C and the temperature Win of the heat medium at the heat medium inlet port of the heat exchanger portion of the artificial lung is $40\pm1$°C.

The blood flow rate is the maximum blood flow rate of the artificial lung. Water is used as the heat medium, and the measurement is performed under the condition in which the flow rate of the heat medium is 10 L/min.

In the calculation of the heat exchange performance coefficient of the artificial lung, the total sum of the performance of heat exchange in the gas exchanger portion and heat exchange in the heat exchanger portion is measured as the heat exchange performance coefficient. Accordingly, the measurement value can be acquired regardless of the type and the configuration of the artificial lung.

3) Blood filling amount of the artificial lung can be measured in units of [mL] using the method described in ISO 7199 5.3.3,

wherein the dry weight of the artificial lung before being filled is measured on a scale. Thereafter, the artificial lung is filled with a physiological salt solution. The weight of the artificial lung after being filled is measured on the scale again, and the filling amount is calculated through the following expression:

$$\text{Filling amount} = (\text{weight after being filled} - \text{dry weight})/(\text{proportion } 1.006 \text{ of physiological salt solution})$$

4) Blood side pressure loss of the artificial lung can be measured using units of [mmHg/(L/min)]

The artificial lung to be measured is embedded in an appropriate circuit, and the pressures at the blood inlet port and the blood outlet port are measured, thereby calculating the pressure loss through the following expression (wherein the blood flow rate is measured at the maximum blood flow rate of the artificial lung to be measured):

```
Pressure   loss=(inlet   port   pressure-outlet   port

pressure)/(maximum blood flow rate)
```

The blood temperature is set to $37\pm1°C$ and the hematocrit value is set to $35\pm1\%$. The test solution is measured by using blood of a cow.

The measurement value can be acquired regardless of the type and the configuration of the artificial lung product.

When the performance factor Fp of commercially available artificial lungs currently on the market is calculated by using the performance factors defined above, the result of Table 1 below is acquired.

[Table 1]

|  | Product A | Product B | Product C | Product D |
|---|---|---|---|---|
| Fp | 1.0 | 1.0 | 0.5 | 1.2 |

Other artificial lungs disclosed in Examples 1 to 3 are described in greater detail below. The gas exchange performance of the artificial lung [L/min], the heat exchange performance coefficient of the artificial lung [absolute scalar number], the blood filling amount of artificial lung [mL] (1 mL=1 L/1,000), and the blood side pressure loss of artificial lung [mmHg/(L/min)] are measured through the measurement method disclosed above and Fp is calculated, thereby acquiring the result of Table 2 below. The artificial lungs of Examples 1 to 3 use artery filtering for internally mounted-type artificial lungs. However, in the method of calculating the performance factor of the present invention, regardless of the form of internally mounting or externally mounting the artery filter, the performance factor can be calculated, evaluated, and compared to each other. Therefore, the method can be applied to various types of artificial lungs. In other words, as described in the measurement method above, in a case where the artery filter is internally mounted, the gas exchange performance of the artificial lung, the heat exchange performance coefficient of the artificial lung, and the blood side pressure loss of the artificial lung are the results reflecting the internally mounted state. Regarding the blood filling amount of the artificial lung, the portion of the artery filter can be relatively ignored with respect to the total blood filling amount. Therefore, the measurement can be performed whether or not the artery filter is internally mounted.

[Table 2]

| Item | Unit | Example 1 | Example 2 | Example 3 |
|---|---|---|---|---|
| Gas exchange performance | L/min | 8.2 | 8.2 | 8.2 |
| Heat exchange performance coefficient | - | 0.58 | 0.57 | 0.59 |
| Blood side pressure loss | mmHg/(L/min) | 15.7 | 15.7 | 15.7 |
| Blood filling amount | mL | 150 | 160 | 180 |
| Performance factor Fp | - | 2.0 | 1.9 | 1.7 |

According to the result of Table 2, it is verified that the artificial lung having the performance factor of Fp of the present invention ranging from 1.5 to 2.5 and preferably ranging from 1.7 to 2.1 has a sufficiently high performance factor compared to the performance factor of conventional artificial lungs available on the market, and when the performance factor of Fp is used during design and evaluation of an artificial lung, a generally excellent artificial lung can be designed, and the performance of the artificial lung can be appropriately evaluated.

2. Calculation of Fp1

<Simplified Performance Factor of Artificial Lung in Entirety>

As an alternative performance factor which can be used for design and evaluation of an artificial lung through simplified measurement and calculation, it is possible to examine a performance factor Fp1 having only the gas exchange performance of the artificial lung [L/min] as the numerator and having the blood filling amount of the artificial lung [L]$\times$ the blood side pressure loss of artificial lung [mmHg/(L/min)] as the denominator. Fp1 can be especially useful in a case where there is a little difference in the heat exchange performance coefficients of the artificial lungs disclosed in Examples 1 to 3 as shown in Table 2. The expression of Fp1 is as follows:

$$Fp1=(gas\ exchange\ performance\ of\ artificial\ lung\ [L/min])/(blood\ filling\ amount\ of\ artificial\ lung\ [L]\times blood\ side\ pressure\ loss\ of\ artificial\ lung\ [mmHg/(L/min)]).$$

According to the result of Table 3, it can be understood that an artificial lung having Fp1 equal to or greater than 2.5 and preferably equal to or greater than 3.0 has a sufficiently high performance factor compared to the performance factor of the artificial lungs available on the market, and when the performance factor of Fp1 is used in design and evaluation of the artificial lung, a generally excellent artificial lung can be designed, and the performance of the artificial lung can be appropriately evaluated through the simplified measurement and calculation.

[Table 3]

| Item | Unit | Example 1 | Example 2 | Example 3 |
|---|---|---|---|---|
| Gas exchange performance | L/min | 8.2 | 8.2 | 8.2 |
| Blood side pressure loss | mmHg/(L/min) | 15.7 | 15.7 | 15.7 |
| Blood filling amount | mL | 150 | 160 | 180 |
| Performance factor Fp1 | - | 3.5 | 3.3 | 2.9 |

3. Calculation of Fp2

<Examination with Only Gas Exchanger Portion>

Regarding the artificial lungs of Examples 1 to 3 of the present invention, when an examination is performed while having only the gas exchanger portion as the subject, since the hollow fiber membrane bundles of Examples 1 to 3 have the same design, a performance factor Fp2 is examined while having gas exchange performance of gas exchanger portion [L/min] as the numerator and blood filling amount of gas exchanger portion [L]×blood side pressure loss of gas exchanger portion [mmHg/(L/min)] as the denominator:

$$Fp2=gas\ exchange\ performance\ of\ gas\ exchanger\ portion\ [L/min]/(blood\ filling\ amount\ of\ gas\ exchanger\ portion\ [L]\times blood\ side\ pressure\ loss\ of\ gas\ exchanger\ portion\ [mmHg/(L/min)])$$

The blood filling amount of the gas exchanger portion indicated herein is calculated based on the volume of only the external circulation blood portion of the gas exchanging hollow fiber membrane bundle disposed in the artificial lung 5, and the blood side pressure loss of the gas exchanger portion is calculated by dividing the pressure difference between blood flowing into the gas exchanger portion and blood flowing out from the gas exchanger portion, by the maximum blood flow rate. According to Fp2, only the gas exchanger portion is taken as the subject, a gas exchanger portion excellent in the gas exchange performance can be designed, and the performance of the gas exchanger portion can be appropriately evaluated. Therefore, as a result, it is possible to design and evaluate an excellent artificial lung.
Description will be given regarding a method of measuring each of the factors for calculating the performance factor while focusing on only the gas exchanger portion of the artificial lung.
For the exchange performance of gas exchanger portion, the same measurement method and the same measurement values as those of the above-described gas exchange performance of the artificial lung in its entirety are used.
For the blood filling amount of the artificial lung with only gas exchanger portion: the unit [mL] (1 mL=1 L/1,000) and the diameter of the hollow fiber membrane bundle of the gas exchanger portion are measured. Then, the square of the radius of hollow fiber membrane×circular constant×length of hollow fiber=occupying volume of hollow fiber can be calculated, and a portion (1-ρ) is calculated based on the occupying ratio (ρ) of the hollow fiber inside the hollow fiber membrane bundle as the blood filling volume. The length of the hollow fiber is obtained through diameter of hollow fiber membrane×circular constant×length of hollow fiber=entire surface area of hollow fiber.

Blood side pressure loss of the gas exchanger portion uses the unit [mmHg/(L/min)]

The pressures at the blood inlet port and the blood outlet port of the gas exchanger portion are measured, and the pressure loss is calculated through the following expression. The blood flow rate is measured at the maximum blood flow rate of the artificial lung to be measured:

```
Pressure   loss=(inlet   port   pressure-outlet   port

pressure)/(maximum blood flow rate).
```

The blood temperature is set to 37±1°C and the hematocrit value is set to 35±1%. The test solution is measured by using blood of a cow.

[Table 4]

| Item | Unit | Examples 1 to 3 |
|---|---|---|
| Gas exchange performance | L/min | 8.2 |
| Blood side pressure loss | mmHg/(L/min) | 10.7 |
| Blood filling amount | mL | 55 |
| Performance factor Fp2 | - | 14.0 |

Meanwhile, when the performance factor Fp2 of the artificial lung with only the gas exchanger portion currently on the market is calculated by using the similar performance factor Fp2, the result of Table 5 below is acquired.

[Table 5]

| | Product A | Product B | Product C |
|---|---|---|---|
| Fp2 | 7.6 | 6.1 | 4.9 |

According to the results of Tables 4 and 5, the artificial lung of which Fp2 is equal to or greater than 8, is preferably equal to or greater than 10, and is more preferably equal to or greater than 12 has a sufficiently high performance factor compared to the product in the related art. When the performance factor Fp2 of the present invention is used, the performance can be appropriately designed and evaluated while having only the gas exchanger portion of the artificial lung as the subject. Therefore, as a result, it is possible to design and evaluate an artificial lung provided with a gas exchanger portion having excellent performance. Therefore, when the gas exchanger portion is designed and manufactured by using the performance factor Fp2, the artificial lung can be configured by combining portions other than the gas exchanger portion with known instruments.

[Examples]

The present invention will be specifically described below by using examples configured in a manner that satisfies the performance factors in the manner indicated. However, the present invention is not limited to the artificial lung and the manufacturing method of these examples.

(Examples 1 to 3)

In Examples 1 to 3, in order to design an artificial lung having a performance factor Fp more excellent than the performance factor Fp of the artificial lung currently on the market, shown in Table 1, the below-described improvement of the gas exchanger portion and the heat exchanger portion is designed and specified.
A gas exchanging hollow fiber is reduced in diameter, and the disposition of the hollow fiber membrane bundle is examined. In addition, a heat exchanging resin tube is employed as the heat exchanging element, and the material thereof and the heat-transfer area thereof are adjusted, thereby examining the disposition of the heat exchanging resin tube. The details are disclosed in Table 6.

The blood filling amount of the artificial lung in its entirety is designed so to be preferably equal to or smaller than 200 mL and to be more preferably equal to or smaller than 190 mL. The blood filling amount with only the gas exchanger portion is designed so to be preferably equal to or smaller than 65 mL and to be more preferably equal to or smaller than 60 mL.

All of Examples 1 to 3 adopt artery filter internally mounted-type artificial lungs. In this case, the blood filling amount of a portion of the artery filter can be ignored with respect to the total blood filling amount. Therefore, the performance factors calculated in Examples 1 to 3 are not influenced by the artery filter.

As a result of the examinations, each of the elements with respect to blood becomes highly dense and an artificial lung having a low blood filling amount and high performance can be designed and evaluated.

[Table 6]

| Item | Example 1 | Example 2 | Example 3 |
|---|---|---|---|
| Hollow fiber bundle | Small diameter polypropylene hollow fiber bundle | Small diameter polypropylene hollow fiber bundle | Small diameter polypropylene hollow fiber bundle |
| Gas exchange area | 1.9 m$^2$ | 1.9 m$^2$ | 1.9 m$^2$ |
| Gas exchanger portion | Hollow fiber bundle winding | Hollow fiber bundle winding | Hollow fiber bundle winding |
| Heat exchange element | Resin tube | Resin tube | Resin tube |
| Heat-transfer area | 0.49 m$^2$ | 0.44 m$^2$ | 0.6 m$^2$ |
| Heat exchanger portion | winding | Bamboo blind winding | Bamboo blind winding |
| Presence of artery filter internally mounted | Present | Present | Present |

Industrial Applicability

An artificial lung of the present invention, regardless of the difference of the type and the structure of the details thereof, gas exchange performance, a heat exchange performance coefficient, a blood filling amount, and a blood side pressure loss are measured and performance factors of the artificial lung can be acquired through combinations of the measured results. The artificial lung can be generally evaluated and compared through the acquired performance factors. Therefore, it is possible to generally evaluate and compare the artificial lungs suitable for various types of methods and structure such as a pediatric artificial lung, an extracorporeal circulation circuit used during cardiotomy, a respiration assisting apparatus for a patient of acute pulmonary insufficiency or cardiac insufficiency, and a percutaneous cardiopulmonary assisting apparatus. Thus, it is possible to expect that the present invention is effectively used in designing and manufacturing an artificial lung which can exhibit excellent performance.

Reference Signs List

2 VEIN RESERVOIR
3 BLOOD SUPPLY PUMP
5 ARTIFICIAL LUNG
6 ARTERY FILTER
7 HEAT MEDIUM SUPPLIER
8 HEAT MEDIUM
9 OXYGEN-CONTAINING GAS SUPPLIER
10 OXYGEN-CONTAINING GAS
11 BLOOD OUTLET PORT
12 BLOOD INLET PORT
14 BLOOD REMOVAL (VENOUS BLOOD) LINE

15    HEART
16    GAS EXCHANGER PORTION
17    HEAT EXCHANGER PORTION
20    ARTIFICIAL HEART-LUNG CIRCUIT DEVICE
21    ARTIFICIAL LUNG HOUSING
23    HOLLOW FIBER MEMBRANE BUNDLE
24    AIR BUBBLE REMOVAL MEANS
25    HEAT EXCHANGE BODY
26    HEAT EXCHANGING PIPE BODY
41    FILTER MEMBER
42    EXHAUST HOLLOW FIBER MEMBRANE LAYER

**Claims**

1.  An artificial lung (5) comprising:

    a gas exchanger portion (16) that is provided with an external circulation-type gas exchanging hollow fiber membrane bundle (23); and
    a heat exchanger portion (17) that is provided with at least a heat exchanging element,
    wherein the artificial lung (5) is designed such that when gas exchange performance of the artificial lung (5), a blood filling amount of the artificial lung (5), and a blood side pressure loss of the artificial lung (5) are measured, and when Fp1=gas exchange performance of artificial lung [L/min]/(blood filling amount of artificial lung [L]×blood side pressure loss of artificial lung [mmHg/(L/min)]) is calculated, a value of Fp1 is equal to or greater than 2.5.

2.  An artificial lung (5) comprising:
    a gas exchanger portion (16) that is provided with an external circulation-type gas exchanging hollow fiber membrane bundle (23); and
    a heat exchanger portion (17) that is provided with at least a heat exchanging element,
    wherein the artificial lung (5) is designed such that when gas exchange performance of the artificial lung (5), a heat exchange performance coefficient of the artificial lung (5), a blood filling amount of the artificial lung (5), and a blood side pressure loss of the artificial lung (5) are measured, and when Fp=(gas exchange performance of artificial lung [L/min]×heat exchange performance coefficient of artificial lung)/(blood filling amount of artificial lung [L]×blood side pressure loss of artificial lung [mmHg/(L/min)]) is calculated, a value of Fp ranges from 1.5 to 2.5.

3.  An artificial lung (5) comprising:

    a gas exchanger portion (16) that is provided with an external circulation-type gas exchanging hollow fiber membrane bundle (23),
    wherein the artificial lung (5) is designed such that when gas exchange performance of the gas exchanger portion (16), a blood filling amount of the gas exchanger portion (16), and a blood side pressure loss of the gas exchanger portion (16) are measured, and when Fp2=gas exchange performance of gas exchanger portion [L/min]/(blood filling amount of gas exchanger portion [L]×blood side pressure loss of gas exchanger portion [mmHg/(L/min)]) is calculated, a value of Fp2 is equal to or greater than 10.

4.  The artificial lung (5) according to any one of Claims 1 to 3,
    wherein the gas exchange performance is a blood flow rate at which blood having oxygen saturation of 97.5% can be acquired in a case where the artificial lung (5) is operated with hemoglobin concentration of 12.0 g/dL and oxygen saturation of 50% in venous blood.

5.  An artificial lung (5) comprising:
    the gas exchanger portion (16) according to Claim 3; and
    a heat exchanger portion (17).

6.  An artificial heart-lung circuit device (20) comprising:
    the artificial lung (5) according to any one of Claims 1 to 5.

**Patentansprüche**

1. Künstliche Lunge (5), umfassend:

   einen Gasaustauscherabschnitt (16), der mit einem externen, gasführenden Hohlfasermembranbündel (23) vom Zirkulations-Typ versehen ist; und
   einen Wärmetauscherabschnitt (17), der mindestens ein Wärmetauschelement aufweist,
   wobei die künstliche Lunge (5) so ausgelegt ist, dass sich bei Messen einer Gasaustauschleistung der künstlichen Lunge (5), einer Blutfüllmenge der künstlichen Lunge (5) und eines blutzuführseitigen Druckverlustes der künstlichen Lunge (5), und bei einer Berechnung von Fp1 = Gasaustauschleistung der künstlichen Lunge [L/min] / (Blutfüllmenge der künstlichen Lunge [L] x blutzuführseitiger Druckverlust der künstlichen Lunge [mmHg/(L/min)]), ein Wert von Fp1 größer gleich 2,5 ergibt.

2. Künstliche Lunge (5), umfassend:

   einen Gasaustauscherabschnitt (16), der mit einem externen, gasförmigen, Hohlfasermembranbündel (23) vom Zirkulations-Typ versehen ist; und
   einen Wärmetauscherabschnitt (17), der mindestens ein Wärmetauschelement aufweist,
   wobei die künstliche Lunge (5) so ausgelegt ist, dass beim Messen einer Gasaustauschleistung der künstlichen Lunge (5), einer Kennzahl der Wärmetauschleistung der künstlichen Lunge (5), einer Blutfüllmenge der künstlichen Lunge (5) und eines blutzuführseitigen Druckverlustes der künstlichen Lunge (5), und bei einer Berechnung von Fp = (Gasleistung der künstlichen Lunge [L/min] x Kennzahl der Wärmetauschleistung der künstlichen Lunge) / (Blutfüllmenge der künstlichen Lunge [L] x blutzuführseitiger Druckverlust der künstlichen Lunge [mmHg/(L/min)]), ein Wert von Fp im Bereich von 1,5 bis 2,5 liegt.

3. Künstliche Lunge (5), umfassend:

   einen Gastauschabschnitt (16), der mit einem externen, gasförmigen, Hohlfasermembranbündel (23) vom Zirkulations-Typ versehen ist,
   wobei die künstliche Lunge (5) so ausgelegt ist, dass sich beim Messen einer Gasaustauschleistung des Gasaustauscherabschnitts (16), einer Blutfüllmenge des Gasaustauscherabschnitts (16) und eines blutzuführseitigen Druckverlustes des Gastauschabschnitts (16), und bei einer Berechnung von Fp2 = Gasaustauschleistung des Gasaustauscherabschnitts [L/min] / (Blutfüllmenge des Gasaustauscherabschnitts [L] x blutzuführseitiger Druckverlust des Gasaustauscherabschnitts [mmHg/(L/min)]), ein Wert von Fp2 größer gleich 10 ergibt.

4. Künstliche Lunge (5) nach einem der Ansprüche 1 bis 3, wobei die Gasaustauschleistung eine Blutflussrate ist, bei der Blut mit einer Sauerstoffsättigung von 97,5% erlangt werden kann, wenn die künstliche Lunge (5) mit einer Hämoglobinkonzentration von 12,0 g/dl und einer Sauerstoffsättigung von 50% in venösem Blut betrieben wird.

5. Künstliche Lunge (5) umfassend:

   den Gasaustauscherabschnitt (16) nach Anspruch 3; und
   einen Wärmetauscherabschnitt (17).

6. Künstliche Herz-Lungen-Kreislaufvorrichtung (20), umfassend:
   die künstliche Lunge (5) nach einem der Ansprüche 1 bis 5.

**Revendications**

1. Poumon artificiel (5) comprenant :

   une partie d'échangeur de gaz (16) qui est pourvue d'un faisceau de membranes à fibres creuses échangeuses de gaz du type à circulation extérieure (23) ; et
   une partie d'échangeur de chaleur (17) qui est pourvue d'au moins un élément d'échange de chaleur,
   dans lequel le poumon artificiel (5) est conçu de telle manière que quand une performance d'échange gazeux du poumon artificiel (5), une quantité de remplissage de sang du poumon artificiel (5), et une perte de pression côté sang du poumon artificiel (5) sont mesurées, et quand Fp1= performance d'échange gazeux du poumon artificiel [L/min]/(quantité de remplissage de sang du poumon artificiel [L]×perte de pression côté sang du poumon artificiel [mmHg/(L/min)]) est calculée, une valeur de Fp1 est supérieure ou égale à 2,5.

2. Poumon artificiel (5) comprenant :

une partie d'échangeur de gaz (16) qui est pourvue d'un faisceau de membranes à fibres creuses échangeuses de gaz du type à circulation extérieure (23) ; et
une partie d'échangeur de chaleur (17) qui est pourvue d'au moins un élément d'échange de chaleur,
dans lequel le poumon artificiel (5) est conçu de telle manière que quand la performance d'échange gazeux du poumon artificiel (5), un coefficient de performance d'échange de chaleur du poumon artificiel (5), une quantité de remplissage de sang du poumon artificiel (5), et une perte de pression côté sang du poumon artificiel (5) sont mesurés, et quand Fp=(performance d'échange gazeux du poumon artificiel [L/min]×coefficient de performance d'échange de chaleur du poumon artificiel)/(quantité de remplissage de sang du poumon artificiel [L]×perte de pression côté sang du poumon artificiel [mmHg/(L/min)]) est calculée, une valeur de Fp est comprise entre 1,5 et 2,5.

3. Poumon artificiel (5) comprenant :

une partie d'échangeur de gaz (16) qui est pourvue d'un faisceau de membranes à fibres creuses échangeuse de gaz du type à circulation extérieure (23),
dans lequel le poumon artificiel (5) est conçu de telle manière que quand un performance d'échange gazeux de la partie d'échangeur de gaz (16), une quantité de remplissage de sang de la partie d'échangeur de gaz (16), et une perte de pression côté sang de la partie d'échangeur de gaz (16) sont mesurées, et quand Fp2=performance d'échange gazeux de la partie d'échangeur de gaz [L/min]/(quantité de remplissage de sang de la partie d'échangeur de gaz [L]×perte de pression côté sang de la partie d'échangeur de gaz [mmHg/(L/min)]) est calculée, une valeur de Fp2 est supérieure ou égale à 10.

4. Poumon artificiel (5) selon l'une quelconque des revendications 1 à 3,
dans lequel la performance d'échange gazeux est un débit sanguin auquel du sang ayant une saturation en oxygène de 97,5% peut être obtenu dans un cas où le poumon artificiel (5) est utilisé avec une concentration en hémoglobine de 12,0 g/dL et une saturation en oxygène de 50% dans le sang veineux.

5. Poumon artificiel (5) comprenant :

la partie d'échangeur de gaz (16) selon la revendication 3 ; et
une partie d'échangeur de chaleur (17).

6. Dispositif de circuit cœur-poumon artificiel (20) comprenant :
le poumon artificiel (5) selon l'une quelconque des revendications 1 à 5.

FIG.1A

HEAT
EXCHANGING
PIPE BODY    26    HEAT
EXCHANGER    17
PORTION

HOLLOW FIBER
MEMBRANE    23    GAS EXCHANGER    16
BUNDLE    PORTION

ARTIFICIAL LUNG
HOUSING    21

FIG.1B

24

HOLLOW FIBER
MEMBRANE BUNDLE    23    41  42

GAS
EXCHANGER
PORTION
16

HEAT
EXCHANGER
PORTION
17

HEAT EXCHANGE BODY  25

FIG.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014514963 T
- US 2014030146 A1
- JP H06237992 A
- US 5706889 A

**Non-patent literature cited in the description**

- **NOGAWA.** Examination of Artificial Lung Performance Evaluation Method. *Artificial Organ,* 1987, vol. 16 (1), 654-657